Europäisches Patentamt

(19) European Patent Office

Office européen des brevets

(11) EP 0 597 007 B1

(12) **EUROPEAN PATENT SPECIFICATION**

(45) Date of publication and mention
of the grant of the patent:
**16.10.1996 Bulletin 1996/42**

(21) Application number: **92916973.8**

(22) Date of filing: **24.07.1992**

(51) Int. Cl.⁶: **A61K 9/107**

(86) International application number:
**PCT/US92/06179**

(87) International publication number:
**WO 93/02664 (18.02.1993 Gazette 1993/05)**

(54) **W/O MICROEMULSIONS**

W/O MIKROEMULSIONEN

MICRO-EMULSION HUILEUSE

(84) Designated Contracting States:
**AT BE CH DE DK ES FR GB GR IT LI LU MC NL
SE**

(30) Priority: **26.07.1991 US 736354**

(43) Date of publication of application:
**18.05.1994 Bulletin 1994/20**

(73) Proprietor: **SMITHKLINE BEECHAM
CORPORATION
Philadelphia Pennsylvania 19101 (US)**

(72) Inventor: **CONSTANTINIDES, Panayiotis
Pericleous
Devon, PA 19333 (US)**

(74) Representative: **Thompson, Clive Beresford
SmithKline Beecham plc
Corporate Intellectual Property,
Two New Horizons Court
Brentford, Middlesex TW8 9EP (GB)**

(56) References cited:
**EP-A- 0 278 660            FR-A- 1 603 312
US-A- 4 840 660**

• **PATENT ABSTRACTS OF JAPAN & JP-A-
55017328 (Tanabe Seiyaku Co. Ltd.) 6 February
1980**

## Description

This invention relates to pharmaceutically acceptable water-in-oil (w/o) self-emulsifying microemulsions containing therapeutic agents, processes for their preparation and their use.

Microemulsions can be defined in general as thermodynamically stable, isotropically clear dispersions of two immiscible liquids stabilized by interfacial films of surface-active molecules. The formation of microemulsions usually involves a combination of three to five components, namely, an oil, water, a surfactant, a cosurfactant and an electrolyte. The tendency towards a water-in-oil (w/o) or an oil-in-water (o/w) microemulsion is dependent on the properties of the oil and the surfactant. Surfactants are conveniently classified on an empirical scale known as the hydrophilic-lipophilic balance (HLB) which runs from 1 to 20. In general, (w/o) microemulsions are formed using surfactants (or emulsifiers) which have an HLB value in the range of about 3 to 6 whilst (o/w) microemulsions are formed using surfactants which have an HLB value in the range of about 8 to 18. It has long been recognized that low interfacial tension contributes to the thermodynamic stability of microemulsions. To achieve this, the surfactant should preferably exhibit low solubility in both the oil and water phases, and be preferentially absorbed at the water-oil interface with concomitant lowering of interfacial tension. When interfacial tension is less than $2 \times 10^{-7}$ N/cm ($2 \times 10^{-2}$ dyn/cm), a stable microemulsion can form. General reviews of microemulsions are provided by Bhargava *et al.*, Pharm. Tech., 46-53, March 1987 and Kahlweit, Science, **240**, 617-621, 1988.

Microemulsions are typically substantially non-opaque, i.e. are transparent or opalescent when viewed by optical microscopic means. In the undisturbed state, they are optically isotropic (non-birefringent) when examined under polarized light. The dispersed phase typically comprises particles or droplets which are normally between 5 and 200 nm in size and this gives rise to their optical transparency. These particles may be spherical although other structures are feasible.

The role of the cosurfactant, usually a short-chain alcohol, is to increase the interfacial fluidity by penetrating the surfactant film and consequently creating a disordered film due to the void space among surfactant molecules. The use of a cosurfactant in microemulsions is however optional and alcohol-free self-emulsifying emulsions and microemulsions have been described in the literature (see for instance, Pouton et al., Int. Journal of Pharmaceutics, **27**, 335-348, 1985 and Osborne *et al.*, J. Disp. Sci. Tech., **9**, 415-423, 1988).

There are many advantages to the use of a microemulsion over a conventional emulsion (or macroemulsion) for drug transport (delivery). Microemulsions form spontaneously, without the need for a high input of energy and are therefore easy to prepare and scale up for commercial applications; they have thermodynamic stability due to their small particle size and therefore have a long shelf life; they have an isotropically clear appearance so that they may be monitored by spectroscopic means; they have a relatively low viscosity and are therefore easy to transport and mix; they have a large interfacial area which accelerates surface reactions; they have a low interfacial tension which permits flexible and high penetrating power and, lastly, they offer the possibility of improved drug solubilization and protection against enzymatic hydrolysis. In addition, microemulsions may undergo phase inversion upon addition of an excess of the dispersed phase or in response to a temperature change and this is a property of these systems that can affect drug release from microemulsions both *in vitro* and *in vivo*. The reasons for this improved drug delivery are not however well understood.

Lipid-based microemulsions have already been proposed to enhance the bioavailability of different drugs, including peptides. Thus, GB 2 222 770A (Sandoz Ltd) describes microemulsions and corresponding microemulsion "pre-concentrates" for use with the highly hydrophobic cyclosporin peptides. Thus, a suitable pre-concentrate comprises 1,2-propylene glycol as the hydrophilic component, a caprylic-capric acid triglyceride as the lipophilic component and a mixture of a polyoxyethylene glycolated hydrogenated castor oil and glycerin monooleate (ratio 11:1) as the surfactant-cosurfactant. Such formulations may then be diluted with water but to give an oil-in-water rather than a water-in-oil microemulsion.

In addition, GB 2 098 865A (Sandoz Ltd) describes topical compositions in the form of microemulsions comprising a water-immiscible organic solvent, an emulsifier, a co-emulsifier, water and a (non-peptide) therapeutic agent. These formulations are said to have improved skin penetrating properties. Suitable organic solvents include ($C_{10-22}$)-fatty acid esters of ($C_{3-18}$)-alcohols such as hexyl laurate, ($C_{12-32}$)-hydrocarbons such as squalene and mono- or diesters of glycerol with a ($C_{6-22}$) carboxylic acid such as glyceryl caprylate (which may also act as a co-emulsifier). There is however no mention of using a medium-chain fatty acid triglyceride as the oil.

Furthermore, US 4 712 239 (Muller *et al.*) describes multi-component systems for pharmaceutical use comprising an oil, a nonionic surfactant with an HLB value above 8 and a co-surfactant which is a partial ether or ester of a polyhydroxyl alcohol and a ($C_{6-22}$) fatty alcohol or acid, which components form a "single phase" on mixing. The special properties of the system are attributed to the particular blend of surfactant and co-surfactant selected. An aqueous phase is an optional extra and the therapeutic agent may be lipophilic or hydrophilic. Such systems are said to give enhanced transdermal delivery characteristics. Amongst the examples provided, one (example 1, formulation I) has PEG (20 EO)-oleic acid glycerol partial esters (40%), caprylic-capric acid glycerol partial esters (42% monoglyceride content, 24%), medium-chain triglycerides (16%) and water (20%). It is to be noted that the ratio of the medium-chain triglyceride to the caprylic-capric acid glycerol partial esters is 1:1.5. In comparison example 1, this is formulated with the drug arecaidine *n*-propyl ester HCl salt. A further example (example 1, formulation VII) incorporates a macromolecule, the polypeptide hirudin but in this, the oil is *iso*-propyl palmitate.

Finally, WO 88/00059 (Engström et al., and the corresponding paper, J. Dispersion Sci. Technol., 11, 479, 1990) discloses controlled release compositions for biologically active materials comprising an "L2-phase" and containing an unsaturated ($C_{16-22}$)-fatty acid monoglyceride and an unsaturated ($C_{16-22}$)-fatty acid triglyceride; in a ratio of from 1:1 to 3:1, and a polar liquid such as water. Such an unsaturated ($C_{16-22}$)-fatty acid monoglyceride is a low HLB surfactant and there is no mention of the additional inclusion of a high HLB surfactant. In addition, a long-chain rather than a medium-chain derivative is used. The existence of an L2 phase had previously been described for a water/monocaprylin/tricaprylin system by Friberg et al., J. Amer. Oil Chem. Soc., **47**, 149, 1970. Again, there is no mention of the additional inclusion of a high HLB surfactant.

EP-A-0 278 660 and FR-A-1 603 312 disclose microemulsions.

We have now surprisingly found that further improved drug delivery characteristics may be obtained using (w/o) microemulsions having a lipophilic phase having certain relative amounts of medium-chain fatty acid triglycerides and low HLB surfactant, in combination with a high HLB surfactant and an aqueous-based hydrophilic phase.

Accordingly, the present invention provides a pharmaceutically acceptable, stable, self-emulsifying water-in-oil (w/o) microemulsion comprising:

(a) a lipophilic phase having a medium-chain fatty acid triglyceride and a low HLB surfactant and in which the ratio of the medium-chain fatty acid triglyceride to the low HLB surfactant is from 5:1 to 1.5:1;
(b) a high HLB surfactant;
(c) an aqueous hydrophilic phase; and
(d) a water-soluble therapeutic agent.

The accompanying drawings contain the following figures:

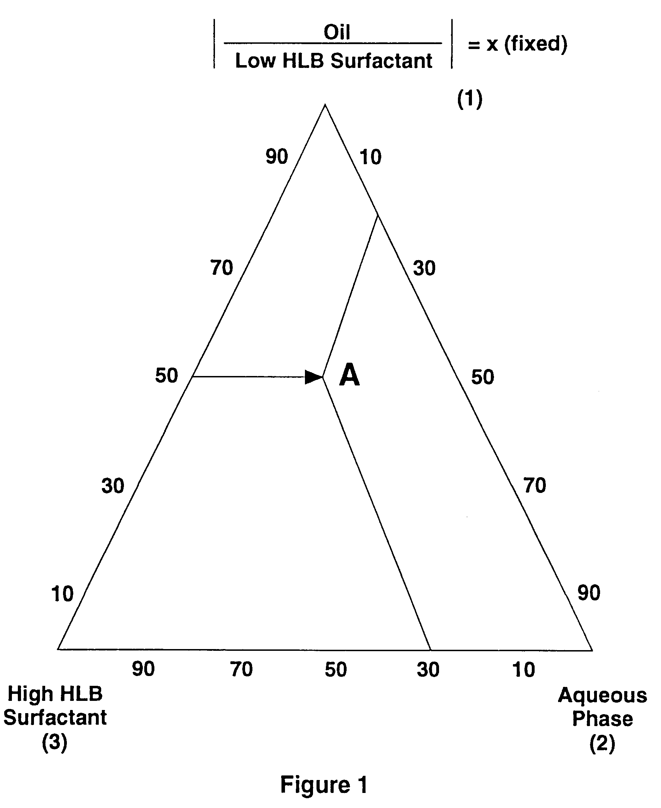

Figure 1 illustrates a pseudo-ternary phase diagram reading of a microemulsion system containing an oil and a low HLB surfactant in a fixed ratio X, a high HLB surfactant and an aqueous phase;

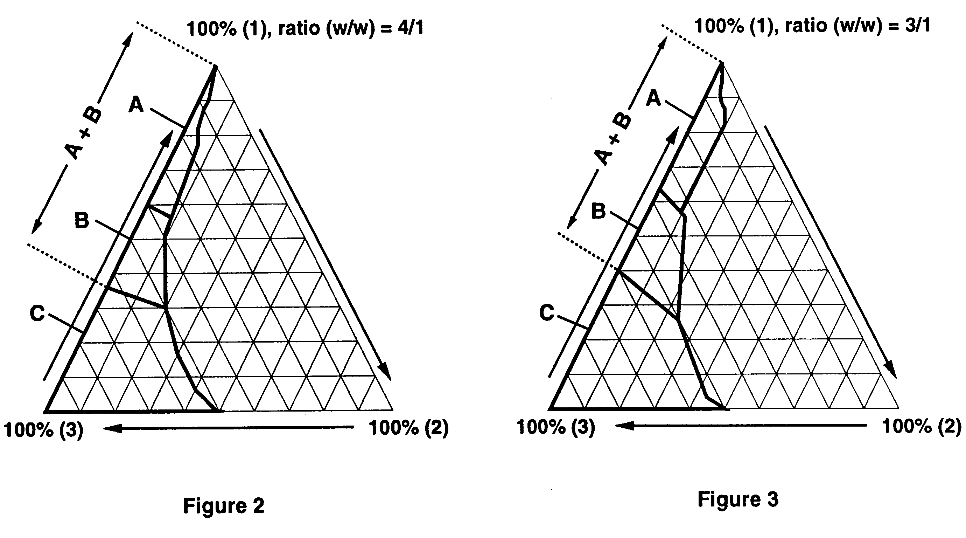

Figure 2 illustrates a pseudo-ternary phase diagram of the microemulsion system Captex 355/Capmul MCM as the oil/low HLB surfactant in a ratio of 4 to 1, labelled as component (1); Tween 80 as the high HLB surfactant, labeled as component (3); and water as the aqueous phase, labeled as component (2);
Figure 3 illustrates a pseudo-ternary phase diagram of the microemulsion system Captex 355/Capmul MCM as the oil/low HLB surfactant in a ratio of 3 to 1, labelled as component (1); Tween 80 as the high HLB surfactant, labeled as component (3); and water as the aqueous phase, labeled as component (2);

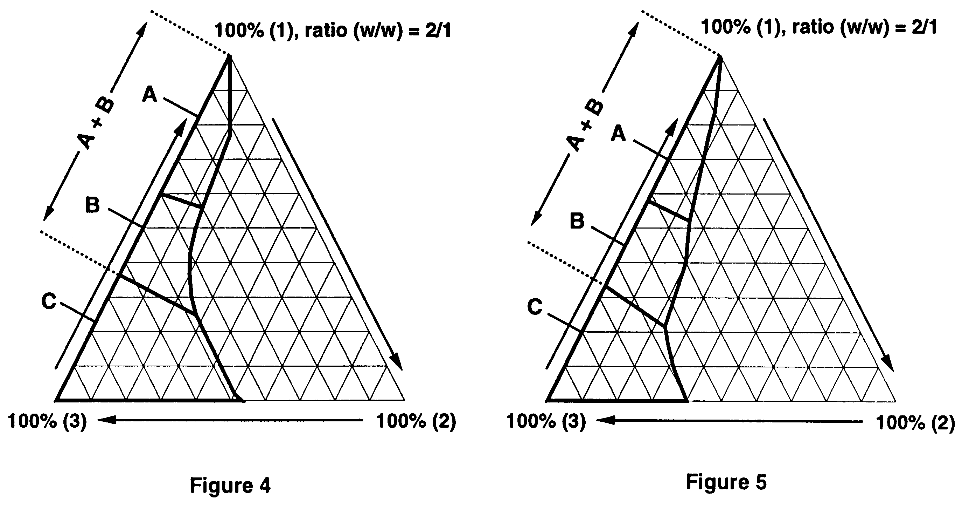

Figure 4 illustrates a pseudo-ternary phase diagram of the microemulsion system Captex 355/Capmul MCM as the oil/low HLB surfactant in a ratio of 2 to 1, labelled as component (1); Tween 80 as the high HLB surfactant, labeled as component (3); and water as the aqueous phase, labeled as component (2); and
Figure 5 illustrates a pseudo-ternary phase diagram of the microemulsion system Captex 8000/Capmul C8 as the oil/low HLB surfactant in a ratio of 2 to 1, labelled as component (1); Tween 80 as the high HLB surfactant, labeled as component (3); and water as the aqueous phase, labeled as component (2).

Suitable medium-chain fatty acid triglycerides for use in the present invention may be of natural, semi-synthetic or synthetic origin and may include blends of different medium chain fatty acid triglycerides. The term "medium-chain fatty acid" as used herein refers to a fatty acid having from 6 to 12, preferably 8 to 10 carbon atoms which may be branched or unbranched, preferably unbranched and which may be optionally substituted. Certain neutral plant oils, such as fractionated coconut oils, provide convenient sources of medium-chain fatty acid triglycerides. The triglyceride suitably comprises from 50 to 100% (w/w) of caprylic ($C_8$) acid and from 0 to 50% (w/w) of capric ($C_{10}$) acid triglycerides. Suitable examples include those available under the trade names MYRITOL; CAPTEX (Karlshams Lipid Specialties, Columbus OH), for instance CAPTEX 355, CAPTEX 300, CAPTEX 350, CAPTEX 850 and CAPTEX 8000; MIGLYOL (BASF), for instance the grades MIGLYOL 810, MIGLYOL 812 and MIGLYOL 818 (which also comprises a linoleic acid triglyceride) and MAZOL 1400 (Mazer Chemical, Gurnee, Il.). The fatty acid content of representative products is : CAPTEX 355™ - caproic acid (2%), caprylic acid (55%) and capric acid (42%); CAPTEX 8000 - at least 98% caprylic acid, MYGOL 810 - caproic acid (2%), caprylic acid (65-75%), capric acid (25-35%) and MIGLYOL 812 - caproic acid (3%), caprylic acid (50-65%), capric acid (30-45%) and lauric acid (5%) (manufacturer's data).

Suitable low HLB surfactants for use in the present invention include medium-chain fatty acid monoglycerides and diglycerides, as well as mixtures thereof, and may also comprise a small amount by weight of free medium-chain fatty acid. The mono- and di-glycerides may each include blends of different medium chain fatty acid mono- and di-glycerides. Suitable medium chain fatty acid mono- and di-glycerides are formed from caprylic and capric acids. Suitable blends comprise from about 50 to 100% caprylic acid and from about 0 to 50% capric acid mono/diglycerides.

Suitable commercial sources of these include the products available under the trade name CAPMUL (Karlsham Lipid Specialties, Columbus OH),for instance the products CAPMUL MCM which comprises monoglycerides (77.4%), diglycerides (21%) and free glycerol (1.6%), with a fatty acid composition of caproic acid (3.2%), caprylic acid (66.8%), capric acid (29.6%), lauric acid (0.3%) and palmitic acid (0.1%) and CAPMUL C8 which has monoglycerides (69.9%), diglycerides (26.1%) and free glycerol (4%), with a fatty acid composition which comprises at least 98% caprylic acid (manufacturers data). Suitably the low HLB surfactant will have an HLB value in the range of about 3 to 6. The capric acid and caprylic acid mono- and di-glycerides blends such as CAPMUL MCM have an HLB value of about 5.5 to 6.

The use in a self-emulsifying (w/o) microemulsion according to the present invention of a low HLB surfactant which is a medium-chain fatty acid monoglyceride and/or a medium-chain fatty acid diglyceride as hereinbefore defined and which is a component of the lipophilic phase provides for reduced droplet size and this is believed to aid in the absorption of the therapeutic agent.

Accordingly in a preferred aspect the present invention provides for a microemulsion in which the medium-chain fatty acid triglyceride is a caprylic acid or a blend of caprylic and capric acids triglycerides as hereinbefore defined and in which the low HLB surfactant is a medium-chain fatty acid monoglyceride or diglyceride or a mixture thereof in which the medium-chain fatty acid is caprylic acid or a mixture of caprylic and capric acids as hereinbefore defined, optionally admixed with a small amount of medium-chain fatty acid, in particular, a blend of caprylic acid triglyceride and caprylic acid monoglyceride or diglyceride or mixture thereof.

Suitable high HLB surfactants for use in the present invention include non-ionic surfactants such as (a) polyoxyethylene fatty acid esters, for example polyoxyethylene stearic acid esters of the type available under the trade name MYRJ (ICI Americas, Inc.), for instance the product MYRJ 52 (a polyoxyethylene 40 stearate); (b) polyoxyetheylene-sorbitan fatty acid esters (polysorbates), for example the mono- and tri-lauryl, palmityl, stearyl and oleyl esters, for instance the polyoxyethylene sorbitan monooleates available under the trade name of TWEEN (ICI Americas Inc.), such as TWEEN 20, 21, 40, 60, 61, 65, 80, 81 and 85, of which class TWEEN 80 is especially preferred; (c) polyoxyethylene glycol long-chain alkyl ethers, such as polyoxyethylated glycol lauryl ether; and (d) polyoxyethylene glycol long-chain alkyl esters, such as PEG-monostearate. For use herein, the high HLB surfactant preferably has an HLB value in the range of 13 to 20.

Suitably, the blend of low and high HLB surfactants will have an HLB value in the range of from about 7 to about 15.

As used herein, the term "therapeutic agent" (hereinafter referred to as "drug") refers to any compound which has biological activity, is soluble in the hydrophilic phase and has an HLB value of at least that of the high HLB surfactant used in the formulation, to ensure that the drug is preferentially dissolved in the hydrophilic rather than the lipophilic phase. This includes both peptides and non-peptides. Suitable peptides include not only small peptides but also larger peptides/polypeptides and proteins. Suitable such peptides preferrably have a molecular weight from about 100 to 10,000, more preferably from about 100 to about 6,000. Especially preferred are peptides having from 2 to 35 amino acid moieties. Higher molecular weight peptides, even those with a molecular weight of above 10,000, up to about 50,000, may also be accomodated in microemulsions of the present invention.

Suitable small peptides have from about 2 to about 10, more preferably from about 2 to about 6 amino acid moieties. Preferred small peptides include the fibrinogen receptor antagonists (RGD containing peptides) which are tetrapeptides with an average molecular weight of about 600. These peptide antagonists are highly potent platelet aggregation inhibitors at plasma levels as low as 1 pmol/ml. Preferred fibrinogen antagonists include the peptide cyclo(S,S)-$N^a$-acetyl-Cys-($N^a$-methyl)Arg-Gly-Asp-Pen-NH$_2$ (Ali et al., EP 0 341 915) and the peptide cyclo(S,S)-(2-mercapto)benzoyl-($N^a$-methyl)Arg-Gly-Asp-(2-mercapto)phenylamide (EP 0 423 212). Other fibrinogen antagonists useful in the present invention are those peptides disclosed by Pierschbacher et al., WO 89/05150 (US/88/04403); Marguerie, EP 0 275 748; Adams et al., U.S. 4,857,508; Zimmerman et al., U.S. 4,683,291; Nutt et al., EP 0 410 537, EP 0 410 539, EP 0 410 540, EP 0 410 541, EP 0 410 767, EP 0 410 833, EP 0 422 937 and EP 0 422 938; Ali et al., EP 0 372 486; Ohba et al., WO 90/02751 (PCT/JP89/00926); Klein et al., U.S. 4,952,562; Scarborough et al., WO 90/15620 (PCT/US90/03417); Ali et al., PCT/US90/06514 and PCT/US92/00999; the peptide-like compounds disclosed by Ali et al., EP 0 381 033 and EP 0 384 362; and the RGD peptide cyclo-$N^a$-acetyl-Cys-Asn-Dtc-Amf-Gly-Asp-Cys-OH (in which Dtc is 4,4'-dimethylthiazolidine-5-carboxylic acid and Amf is 4-aminomethylphenylalanine).

The RGD peptide may be usefully included in the microemulsion formulation in an amount up to about 600mg/g of the hydrophilic phase or from 0.1 to 60 mg/g of the formulation.

Other peptides useful in the present invention include, but are not limited to, other RGD containing peptides such as those disclosed by Momany, U.S. 4,411,890 and U.S. 4,410,513; Bowers et al., U.S. 4,880,778, U.S. 4,880,777, U.S. 4,839,344; and WO 89/10933 (PCT/US89/01829); the peptide Ala-His-D-Nal-Ala-Trp-D-Phe-Lys-NH$_2$ (in which Nal represents β-naphthylalanine) and the peptides disclosed by Momany, U.S. 4,228,158, U.S. 4,228,157, U.S. 4,228,156, U.S. 4,228,155, U.S. 4,226,857, U.S. 4,224,316, U.S. 4,223,021, U.S. 4,223,020, U.S. 4,223,019 and U.S. 4,410,512.

Other suitable peptides include hexapeptides such as the growth hormone releasing peptide (GHRP) His-D-Trp-Ala-Trp-D-Phe-Lys-NH$_2$, (Momany, US 4,411,890). This may usefully be included in an amount up to about 250mg/g of the hydrophilic phase or from 0.1 to 25mg/kg of the formulation.

Suitable larger polypeptides and proteins for use in microemulsions of the present invention include insulin, calcitonin, elcatonin, calcitonin-gene related peptide and porcine somatostatin as well as analogs and homologs thereof. Other suitable larger polypeptides include those disclosed by Pierschbacher *et al.*, U.S. 4,589,881 (>30 residues); Bittle *et al.*, U.S. 4,544,500 (20-30 residues); and Dimarchi *et al.*, EP 0 204 480 (>34 residues).

Other type of compounds useful in the present invention include analogs or homologs of LHRH which display potent LH releasing activity or inhibit the activity of LHRH; analogs or homologs of HP5 which possesses hematopoetic activity; analogs or homologs of endothelin which possess hypotensive activity; analogs or homologs of enkephalin which have antinociceptive activity; analogs or homologs of chlorecystokinnin; analogs or homologs of cyclosporin A which have immunosuppressive activity; analogs or homologs of atrial natriuretic factor; peptidergic antineoplastic agents; analogs or homologs of gastrin releasing peptide; analogs or homologs of somatostatin; gastrin antagonists; bradykinn antagonists; neurotensin antagonists; bombesin antagonists; oxytocin agonists and antagonists; vasopressin agonists and antagonists; hirudin analogs and homologs; analogs and homologs of the cytoprotective peptide-cyclolinopeptide; alpha MSH analogs; analogs, and homologs of MSH releasing factor (Pro-Leu-Gly-NH$_2$); peptides which inhibit collagenase; peptides which inhibit elastase, peptides which inhibit renin; peptides which inhibit HIV protease; peptides which inhibit angiotensin converting enzyme; peptides which inhibit chymases and tryptases and peptides which inhibit blood coagulation enyzmes.

Other suitable drugs include non-peptide therapeutic agents such as antibotics, antimicrobial agents, antineoplastic agents, cardiovascular and renal agents, antiinflammatory, immunosuppressive and immunostimulatory agents and CNS agents.

Preferably, the drug is a peptide such as a fibrinogen receptor antagonist peptide (an RGD peptide), GHRP (His-D-Trp-Ala-Trp-D-Phe-Lys-NH$_2$), a vasopressin, a calcitonin or an insulin, more preferably the fibrinogen receptor antagonist peptides cyclo(S,S)-N$^a$-acetyl-Cys-(N$^a$-methyl)Arg-Gly-Asp-Pen-NH$_2$ or cyclo(S,S)-(2-mercapto)benzoyl-(N$^a$-methyl)Arg-Gly-Asp-(2-mercapto)phenylamide or GHRP.

In a prefered aspect, the present invention provides microemulsions comprising a peptide which may be orally administered and which will retain biological activity, thereby overcoming the disadvantages of earlier formulations in which the bioavailability of the peptide has been less than satisfactory. In particular, the present invention provides formulations which by their nature permit the preparation and administration of a peptide in sufficiently high concentration to allow not only convenient oral administration but also adequate bioavailability of the peptide.

For a water-soluble drug, the degree of incorporation into the (w/o) microemulsions of the present invention is limited only by its solubility in the hydrophilic phase. The ionic strength and pH (within the range 3 to 10) may be adjusted to aid dissolution, without compromising the integrity of the microemulsion.

The aqueous hydrophilic phase suitably comprises water or an isotonic saline solution and may also include a pharmaceutically acceptable solvent which is non-miscible with the selcted lipophilic phase.

In a preferred aspect, it has been found that in microemulsions of the present invention, the use of a mono- or polyhydroxyalcohol co-surfactant, such as ethanol, butanol or propylene glycol, as the major component of the hydrophilic phase may be avoided. This has the advantage of not only mitigating the stability and processing difficulties associated with the use of such but also reducing the concomitant stomach and duodenum irritation. Accordingly, the hydrophilic phase of microemulsions of the present invention may be essentially aqueous and comprise less than 10%, preferably less than 5% and more preferably less than 2% by weight of the phase of an alcohol.

It will be readily appreciated by the skilled person that not all blends of a medium-chain fatty acid triglyceride, low and high HLB surfactants and hydrophilic phase will yield stable, self-emulsifying microemulsions within the scope of the present invention. Appropriate ratios may, however, be readily determined by the skilled man with the aid of a phase diagram such as that illustrated in Fig. 1. As the system comprises four components *viz* a medium-chain fatty acid triglyceride (oil), a low HLB surfactant, a high HLB surfactant and an aqueous/hydrophilic phase, a pseudo-ternary phase diagram is employed. In this, the ratio of two components such as the oil and the low HLB surfactant is kept constant so that there are only three variables, each of which can then be represented by one side of the triangle. Thus, in fig. 1, (1) represents the mixture of the oil and the low HLB surfactant, at a fixed ratio X, (2) the hydrophilic (aqueous) phase and (3) the high HLB surfactant. By way of example, the point "A" represents a mixture 50% oil plus low HLB surfactant, 20% aqueous phase and 30% high HLB surfactant.

The regions of the phase diagram in which microemulsions according to the present invention exist may be determined by titrating a mixture of the oil and low HLB surfactant (in a fixed ratio) against the high HLB surfactant and the hydrophilic phase, noting points of phase separation, turbidity and transparency. Clear, transparent formulations are indicative of the formation of a stable microemulsion. Liquid and gel formulations may be obtained at room temperature according to the specific nature of the components employed.

Once stable transparent systems are obtained, simple tests, such as dye solubilization, dispersibility in water and conductivity measurements may be used to determine whether the microemulsion is an (o/w)- or a (w/o)-type. A water-soluble dye will disperse in an (o/w) microemulsion whilst it will remain in its original form in a (w/o) microemulsion. Likewise, (o/w) microemulsions are generally dispersible in water whereas (w/o) microemulsions are generally not. In addition, (o/w) microemulsions conduct electricity whereas (w/o) do not. The isotropic nature of the system may be confirmed by examination thereof under polarised light. The microemulsions being micellar in nature are isotropic and therefore non-birefringent when examined under polarised light.

From this phase diagram, appropriate percentages may then be read off. The process may then be repeated for other ratios of oil to low HLB surfactant so that an overall picture may be obtained.

Representative pseudo-ternary phase diagram of systems containing a medium-chain oil (CAPTEX 335) and low HLB surfactant (CAPMUL MCM™) (in the ratios 4:1, 3:1 and 2:1), high HLB surfactant (Tween 80) and water are shown as Figures 2, 3 and 4, respectively. The mixture of oil plus the low HLB surfactant is indicated as component (1), water as component (2) and the high HLB surfactant as compontent (3). These systems produces a wide range of clear, transparent microemulsions which are shown in the phase diagram as the microemulsion field (shaded areas) which field may be usefully be sub-divided into regions (A), (B) and (C).

This sub-division is based primarily on differences in conductance, viscosity and dilutability in the presence of excess water (at least 5-fold). Both the viscosity and conductance increase from region (A) to (C), with major changes observed between (B) and (C). In the presence of excess of the dispersed phase (saline or water), microemulsions of regions (A) and (B) are inverted to turbid emulsions (o/w) indicative of their original (w/o) nature. In contrast, microemulsions from region (C) remains clear. Although this may superficially suggest that the microemulsions from region (C) are oil-in-water (o/w) rather than water-in-oil (w/o), it is believed that these too are also water-in-oil. Initial addition of water is thought to cause an immediate inversion to an oil-in-water isotropic system (microemulsion) so that further dilution causes no turbidity. This belief is based on the observed conductivities of microemulsions in region (C) which are extremely low and characteristic of a (w/o) microemulsion. In comparison, an (o/w) microemulsion would be expected to have a much higher conductivity, reflecting the presence of an aqueous continuous phase. Thus, with an aqueous phase of saline (at 3%), the conductance of microemulsions within regions (A), (B) and (C) varied between 0.5 and 4 $\mu$mho whereas the conductance of the same saline solution *per se* was 13,400 $\mu$mho.

The calculated final HLB values for the blend of low and high HLB surfactants in the regions (A), (B) and (C) are 7 to 11, 11 to 13 and 13 to 15, respectively.

Microemulsions within the scope of the present invention are those falling within regions (A), (B) and (C) of the pseudo-ternary phase diagram.

Accordingly, in a further aspect the present invention provides stable, self-emulsifying (w/o) microemulsions as hereinbefore defined in which the relative proportions of the various components lie within regions (A), (B) and (C), preferably (A) and (B), more preferably (A), of pseudo-ternary phase diagrams such as Figures 2 to 4.

In general, in the representative system, stable clear, transparent liquid microemulsions were obtained when the oil plus low HLB surfactant was present in the range from about 40% to less than 100% , the high HLB surfactant less than 50% and the water less than 20% (w/w) of the microemulsion.

By this process of constructing a representative range of phase diagrams, it has been possible to determine appropriate quantities of the various components which will lead to stable, self-emulsifying microemulsions falling within the present invention.

Suitably, the medium-chain fatty acid triglyceride and the low HLB surfactant together comprise from about 8 to about 95%, preferably about 10 to about 90%, more preferably about 40 to about 90%, most preferably about 60 to about 90% (w/w) of the microemulsion. The medium-chain fatty acid triglyceride and the low HLB surfactant may be combined and mixed at various ratios. Useful (w/o) microemulsions of relatively low viscosity may be obtained when the ratio of medium-chain fatty acid triglyceride to low HLB surfactant is in the range of about 5:1 to about 1.5:1, preferably about 4:1 to about 2:1. It is found that as the ratio of medium-chain fatty acid triglyceride to low HLB surfactant is increased towards 5:1, region (C) of the microemulsion existence field becomes increasingly predominant.

Suitably, the high HLB surfactant is present in the range of about 5 to about 75%, preferably about 5 to about 50%, more preferably from about 7.5 to about 30% (w/w) of the microemulsion.

Suitably the hydrophilic phase comprises from just greater than 0 to about 40%, preferably from about 0.1 to 20%, more preferably from about 0.1 to 10% and most preferably from about 1 to 5% (w/w) of the microemulsion.

It will be readily appreciated by the skilled person that, in general, an increase in the relative amount of high HLB surfactant will have to be matched by an increase in the relative amount of hydrophilic phase.

In preferred microemulsions of the present invention, the lipophilic phase comprises about 10-95%, preferably 40 to 90%, more preferably 60 to 90%, the high HLB surfactant from about 5 to 90%, preferably from 5 to 50%, more preferably 5 to 30% and the hydrophilic phase less than 40%, preferably less than 10% and more preferably less than 5% (w/w) of the microemulsion. Within such microemulsions, the ratio of medium-chain fatty acid triglyceride to low HLB surfactant is preferably between 4:1 and 2:1.

The microemulsions of the present invention are substantially non-opaque, that is they are transparent or opalescent when viewed by optical microscopic means. In their undisturbed state, they are optically isotropic (non-birefringent) when examined under polarized light. They exhibit excellent stability at low and ambient temperatures, without phase separation, clouding or precipitation, even over prolonged periods of time. The formulations may be stored in a stable form at various temperatures, such as at 4°C, ambient temperature, 37°C and at 50°C, preferably at 4°C or ambient temperatures. Peptide-containing microemulsions of the present invention exhibit a similar stability (shelf life) profile to that of the corresponding peptide-free microemulsions. Stable (w/o) microemulsions may be formed when the pH of the aqueous phase varies from a pH of approximately 3 to about 10, a property that can be beneficial for drugs exhibiting higher solubility at low or high pH. The microemulsions are of varying viscosity, with formulations which are mobile liquids or gels at ambient temperature. Microemulsions with a relatively higher amount of a high HLB surfactant such as TWEEN 80 tend to be more viscous due to the greater viscosity of this material.

Preferably, the diameter of droplets or particles of the microemulsions of the present invention, measured, for instance, as the number-average diameter by laser light scattering techniques, is less than 150 nm, more preferably less than 100 nm, yet more preferably less than 50 nm and most preferably in the range 5 to 35 nm.

The various phases may optionally contain further ingredients, such as, but not limited to:

i) lipids, such as phospholipids, in particular lecithins, such as soya bean lecithins, egg lecithin or egg phosphatide, cholesterol or oleic acid;

ii) antioxidants such as n-propyl gallate, butylated hydroxyanisole (BHA) and mixed isomers thereof, d-a-tocopherol and mixed isomers thereof, ascorbic acid, propylparaben, methylparaben and citric acid (monohydrate);

iii) bile salts, for instance as their alkali metal salts, such as sodium taurocholate;

iv) stabilizers, such as hydroxypropyl cellulose;

v) antimicrobials, such as benzoic acid (sodium salt);

vi) dioctylsuccinate, di-octylsodium sulfosuccinate or sodium lauryl sulfate;

vii) propylene glycol mono-and di-fatty acid esters, such as propylene glycol dicaprylate, dilaurate, hydroxystearate, isostearate, laurate, ricinolate, etc., of which the propylene glycol caprylic/capric acid diesters commercially known as MIGLYOL 840™ are especially preferred; and

viii) protease inhibitors such as aprotinin.

The microemulsions of the present invention form spontaneously or substantially spontaneously when their components are brought into contact, that is without the application of substantial energy supply, for instance in the absence of high shear energy such as imparted by homogenization and/or microfluidization or other mechanical agitation. Accordingly the microemulsions may be readily prepared by the simple process of admixing appropriate quantities, with gentle hand mixing or stirring if necessary to ensure thorough mixing. Preferably, the drug is dissolved in the hydrophilic phase, either directly or by dilution of a stock solution thereof and this may then be added to a pre-mixed combination of the oil and the low HLB surfactant with mixing, followed by the high HLB surfactant or *vice versa*. Alternatively, a drug-free microemulsion may be initially prepared by admixing the oil, the low HLB surfactant, the high HLB surfactant and drug-free hydrophilic phase; to which may then be added further hydrophilic phase in which the drug is dissolved. Whilst higher temperatures (40-60°C) may be needed to solubilize all components during the preparation of the microemulsion, the preferred systems may be formulated at room temperature. Formulation at ambient temperature is particularly advantageous for thermolabile active ingredients such as peptides.

Microemulsions of the present invention are pharmaceutical compositions which comprise a therapeutic agent and are therefore intended for use in therapy, for administration to animals, including man.

It will be recognized by one of skill in the art that the amount of drug required for therapeutic effect on administration will, of course, vary with the agent chosen, the nature and severity of the condition and the animal undergoing treatment, and is ultimately at the discretion of the physician. Furthermore, the optimal quantity and spacing of individual dosages of a drug will be determined by the nature and extent of the condition being treated, the form, route and site of administration, the particular patient being treated and that such optima can be determined by conventional techniques. It will also be appreciated that the optimal course of treatment, that is, the number of doses given, can be ascertained by those skilled in the art using conventional course of treatment determination tests.

The present invention also provides for the use of a medium-chain fatty acid triglyceride, a low HLB surfactant, a high HLB surfactant, a therapeutic agent and a hydrophilic phase as hereinbefore defined in the manufacture of a medicament.

Microemulsions of the present invention may be used for oral, topical, rectal, intra-vaginal or other forms of systemic administration and accordingly will be presented in forms suitable for such. Thus for instance, microemulsions intended for oral administration may be presented in soft gelatin capsules whilst the viscosity characteristics of some of the microemulsions make them suitable for direct topical application. Compositions suitable for oral or topical administration are especially prefered.

The microemulsions of the present invention without a drug are novel and useful as precursors to drug-containing microemulsions. Accordingly, in a further aspect, the present invention provides a stable, self-emulsifying water-in-oil (w/o) microemulsion comprising:
(a) a lipophilic phase having a medium-chain fatty acid triglyceride and a low HLB surfactant and in which the ratio of the medium-chain fatty acid triglyceride to the low HLB surfactant is from 5:1 to 1.5:1; (b) a high HLB surfactant; and (c) an aqueous hydrophilic phase in which each of (a), (b) and (c) are as hereinbefore defined and pharmaceutically acceptable.

The invention will now be illustrated by, but not limited to, the following descriptions (drug-free microemulsions) and examples (drug-containing microemulsions) and biological examples.

## DESCRIPTIONS

### Description 1 - Phase Diagrams for Representative Microemulsions

Pseudo-ternary phase diagrams were constructed for representative systems comprising:

| medium-chain fatty acid triglyceride (oil) | CAPTEX 335 |
| --- | --- |
| low HLB surfactant | CAPMUL MCM |
| high HLB surfactant | TWEEN 80 |
| aqueous phase | water |

in which the ratio of the oil to the low HLB surfactant was 4:1, 3:1 or 2:1.

The regions of the phase diagram in which microemulsions according to the present invention exist were determined by titrating a mixture of the oil and low HLB surfactant (in a fixed ratio) against the high HLB surfactant and the aqueous phase, noting points of phase separation, turbidity and transparency.

The resultant phase diagrams are shown as figures 2, 3 and 4. Due to the relatively low viscosity of the particular oil and the low HLB surfactant, these components were readily formulated at room temperature. A wide range of clear transparent (w/o) microemulsions as shown by regions (A), (B) and (C) were available. These were stable at room temperature.

When examined under polarised light, non-birefringent behaviour was observed. They were observed to have extremely low electrical conductance, in the range 0.5 to 4.0 mhos for a 3:1 ratio of oil to low HLB surfactant, with saline as the aqueous phase.

These phase diagrams show that microemulsions within the scope of the present invention are obtained for ratios of medium chain fatty acid triglyceride to low HLB surfactant ranging from 4:1 to 2:1.

From these, the skilled person will readily appreciate that the microemulsion existence regions for other systems may be readily determined by focussing on the ratios defined by regions (A), (B) and (C) rather than having to repeat the whole process and look at relative amounts well removed from these regions.

Using the same general method, a similar pseudo-ternary phase diagram was constructed using the product CAPTEX 8000 as the oil and the product CAPMUL C8 as the low HLB surfactant, in a ratio of 2:1. The high HLB surfactant was TWEEN 80 and the aqueous phase saline solution. The (w/o) microemulsions obtained with this different combination of oil and low HLB surfactant were similar to those described above. The resultant phase diagram is shown as Fig. 5.

A typical microemulsion from region (A) of Fig. 3 and comprising CAPTEX 335/CAPMUL MCM (ratio 3:1, 87%), TWEEN 80 (10%) and aqueous (3%) was found to have the following physical characteristics:

| Density | 0.9677 |
| --- | --- |
| Refractive Index | 1.449 |
| Viscosity | 56.7cP |
| Conductance | 0.540µmhos |
| Particle size* | 15.2 ± 4.1nm |
| Polydispersibility* | 0.153 |

* both expressed as particle no. results; a latex bead standard of 63 nm had a particle size of 64.2±15.1 and a polydispersibility of 0.031.

**Description 2 - (w/o) microemulsions**

Water-in-oil microemulsions were prepared on a 10g scale using the following:

| | |
|---|---|
| MYGLYOL 812 or MYGLYOL 812 and CAPTEX 335 (1:1) plus CAPMUL MCM (total oil to CAPMUL MCM = 3:1) | 87% |
| TWEEN 80 | 10 |
| Deionised water | 3 |

**EXAMPLES**

For further studies on microemulsions incorporating a drug, an optimal formulation was selected from the centre of region (A) of the phase diagrams hereinbefore described. This formulation had the composition:

| | |
|---|---|
| CAPTEX 335/ CAPMUL MCM (ratio 3:1) | 87.0-87.5% |
| TWEEN 80 | 10 |
| saline solution | 2.5-3 |

These microemulsions were generally formulated by initially preparing the drug-containing hydrophilic phase, either by dissolving the appropriate amount of drug in the appropriate amount of saline solution or, more preferably, using a stock solution which was then further diluted if so required, with vortex stirring if necessary to obtain complete dissolution. The hydrophilic phase containing the drug was then added to the appropriate amounts (by weight) of a mixture of the oil and the low HLB surfactant, to which was then added the high HLB surfactant, with gentle stirring (magnetic hot plate stirrer). Alternatively, the hydrophilic phase containing the drug was added to the high HLB surfactant and following upon complete mixing, this was added to the oil plus low HLB surfactant mixture. If necessary, the drug-containing microemulsion was then diluted with the corresponding drug-free microemulsion to adjust the concentration of the drug. Batches were routinely prepared on a 5 or 10 g scale. In addition larger scale (50 to 500g) batches were also prepared.

Following the standard procedure outlined above, the following drug-containing microemulsions were prepared, as shown in the following table:

## Table of Examples

| Example | Drug | Drug conc. mg/g form. | oil & low HLB surfactant[a] %(w/w) | high HLB surfactant[b] %(w/w) | aqueous phase[c] %(w/w) |
|---------|------|------|------|------|------|
| 1 | RGD peptide[d] | 6 | 87.0 | 10.0 | 3 |
| 2a | GHRP[e] | 1.5 | 87.0 | 10.0 | 3.0 |
| 2b | GHRP[e] | 0.8 | 87.0 | 10.0 | 3.0 |
| 2c | GHRP[e] | 0.8 | 70.0 | 25.0 | 5.0 |
| 2d | GHRP[e] | 0.8 | 50.0 | 40.0 | 10.0 |
| 2e | GHRP[e] | 0.8 | 30.0 | 50.0 | 20.0 |
| 2f | GHRP[e] | 0.8 | 20.0 | 70.0 | 10.0 |
| 2g | GHRP[e] | 0.8 | 87 | 10 | 3 |
| 3 | vaso-pressin[f] | 0.06 | 87.0 | 10.0 | 3.0 |
| 4 | calcitonin[g] | 0.02 | 87.0 | 10.0 | 3.0 |
| 5 | calcitonin[g] | 0.09 | 87.0 | 10.0 | 3.0 |
| 6 | insulin[h] | 0.15 (37 IU) | 87.0 | 10.0 | 3.0 |
| 7 | insulin[h] | 0.38 (92 IU) | 82.5 | 10.0 | 7.5 |
| 8a | AII antag[i] | 0.15 | 87 | 10 | 3 |
| 8b | AII antag[i] | 0.5 | 50 | 40 | 10 |
| 8c | AII antag[i] | 1.0 | 30 | 50 | 20 |
| 9 | calcein[j] | 6.2 | 87 | 10 | 3 |

## Footnotes to table

[a] CAPTEX 335 and CAPMUL MCM in ratio 3:1, apart from 2g which had CAPTEX 8000 and CAPMUL C8 in ratio 2:1;

[b] TWEEN 80;

[c] varied as given below according to drug being used;

[d] cyclo(S,S)-(2-mercapto)benzoyl-($N^a$-methyl)-Arg-Gly-Asp-(2-mercapto)-phenylamide (MW of about 650), aq. = saline;

[e] His-D-Trp-Ala-Trp-D-Phe-Lys-$NH_2$ (MW of about 850), aq. = isotonic soln containing acetic acid and sodium chloride at pH 5.0;

[f] Val-Asp-Cys-Tyr-Phe-Gln-Asn-Cys-Pro-Arg-Gly- $NH_2$ (MW of about 1300) (ICN Biochemicals), aq. = saline;

[g] salmon calcitonin (contains 32 amino acids, MW of about 3500) (ICN Biochemicals), aq. = saline;

[h] polypeptide with a MW of about 6000 (ICN Biochemicals), aq. = phosphate-buffered saline;

[i] angiotensin II antagonist - (E)-a-[[2-butyl-1-[(4-carboxy-1-naphtalenyl)methyl]-1H-imidazol-5-yl]methylene]-2-thiophenepropanoic acid (MW = 475), aq. = 3.5% (w/v) sodium bicarbonate; and

[j] calcein (5(6)-carboxyfluorescein, MW = 623) is widely used as a model water soluble molecule in studies on drug carriers - see T.M. Allen, "Calcein as a tool in liposome methodology", Liposome Technology, G. Gregoriadis, ed., III, 178-182, CRC Press, Boca Raton, 1984, aq. = Tris buffer at pH 7.5 (10ml).

## BIOLOGICAL EXAMPLES

### Biological Example 1- Assessment of GI Irritation

Using standard methodology (Szabo *et al.*, J. Pharmacol. Methods, **13**, 59-66, 1985), a drug-free microemulsion comprising CAPTEX 355 and CAPMUL MCM (ratio 3:1) (87%), TWEEN 80 (10%) and saline (3%) was assessed for its potential to cause GI irritation in rats. After oral dosing (3.3 ml/kg), the mucosal surfaces of both the stomach and duodenum were found to be free of any lesions when examined by the naked eye. Under microscopic examination, however, some light petechial areas were seen on the internal mucosa of the stomach only. After *i.d.* dosing (3.3ml/kg), the entire GI tract was found to be free of any lesions.

### Biological Example 2 - Bioavailability of an RGD Peptide

Using a conventional conscious rat model, the bioavailability of an RGD peptide in the microemulsion of example 1 (comprising 6mg/g microemulsion) was assessed and compared with that of the same peptide administered as an aqueous solution. After *i.d.* dosing at a level of 8.4mg/kg (coresponding to 3.3ml/kg microemulsion), bioavailability was 21.9±5.7% (n=3). In comparison, the bioavailability of the same peptide administered as an aqueous solution was only 0.5%. Thus, by the expedient of formulating the RGD peptide in a microemulsion, an approximately fifty-fold enhancement of bioavailability was obtained.

### Biological Example 3 - Bioavailablity of Calcein

In a similar manner to Biological Example 1, but using an unconscious rat model (Walker *et al.*, Life Sciences, **47**, 29-36, 1990), the bioavailability of the model compound calcein when dosed as the formulation of example 9 was assessed and compared with that obtained when the same compound was dosed by the same route but as a solution in saline. Being a fluorescent compound, the levels of the compound in the plasma samples could be readily determined using fluorescence spectroscopy. After *i.d.* dosing at 3.0umol/kg (1.0ml/kg microemulsion), bioavailability was 17.2±2.4% (n=4). In comparison, the bioavailability of the same peptide administered as a saline solution was only 1.3±0.5% (n=5).

**Biological Example 4 - Demonstration of *in vivo* activity of a drug delivered by a microemulsion**

**(a) RGD peptide**

The *in vivo* activity of the microemulsion of example 1 containing the RGD petide (6mg/g formulation) was demonstrated in a standard platelet aggregation assay using dogs (Samanen *et al.*, Med. Chem., **34**, 3114-3125, 1991). Following oral dosing of the microemulsion containing the peptide (in a gelatin capsule) at 3 mg/kg (0.5 mg/kg microemulsion), inhibition was observed which was, in general, more pronounced and more sustained than that observed in a corresponding control experiment in which the RGD peptide was dosed as a saline solution.

**(b) GHRP**

The *in vivo* activity of the microemulsion of example 2a containing GHRP (1.5mg/g of formulation) was demonstrated in a standard *in vivo* assay for growth hormone (GH) levels in rats (Walker *et al.*, Life Sciences, **47**, 29-36, 1990). Following *i.d.* dosing at 3mg/kg (2.0ml/kg microemulsion), analysis of blood samples indicated significant GH levels (> 30ng/ml in all 5 rats tested). In comparison, samples from the control group which had been treated with a saline solution of GHRP had neglible levels of GH. This data indicated that the GHRP was pharmacologically active when dosed *i.d.* as a microemulsion formulation.

**Claims**

1. A pharmaceutically acceptable, stable, self-emulsifying water-in-oil (w/o) microemulsion comprising:

   (a) a lipophilic phase having a medium-chain fatty acid triglyceride and a low HLB surfactant and in which the ratio of the medium-chain fatty acid triglyceride to the low HLB surfactant is from 5:1 to 1.5:1;
   (b) a high HLB surfactant selected from a polyoxyethylene fatty acid ester, a polyoxyethylene-sorbitan fatty acid ester, a polyoxyethylene glycol long-chain alkyl ether or a polyoxyethylene glycol long-chain alkyl ester; which surfactant is present in the range of 5 to 75% (w/w) of the microemulsion;
   (c) an aqueous hydrophilic phase; and
   (d) a water-soluble therapeutic agent.

2. A microemulsion as claimed in claim 1 in which the triglyceride comprises from 50 to 100% (w/w) of caprylic acid and from 0 to 50% (w/w) of capric acid triglycerides.

3. A microemulsion as claimed in claim 1 or 2 in which the low HLB surfactant is a medium-chain fatty acid monoglyceride or diglyceride or a mixture thereof, optionally comprising a small amount by weight of free medium-chain fatty acid.

4. A microemulsion as claimed in claim 3 in which the medium-chain fatty acid monoglyceride or diglyceride is formed from about 50 to 100% caprylic acid and from about 0 to 50% capric acid.

5. A microemulsion as claimed in any one of claims 1 to 4 in which the therapeutic agent is a peptide having a molecular weight in the range of 100 to 10,000.

6. A microemulsion as claimed in claim 5 in which the peptide is an RGD containing peptide, the peptide GHRP (His-D-Trp-Ala-Trp-D-Phe-Lys-NH$_2$), a fibrinogen receptor antagonist peptide (an RGD peptide), a vasopressin, a calcitonin or an insulin.

7. A microemulsion as claimed in claim 6 in which the RGD peptide is cyclo(S,S)-N$^a$-acetyl-Cys-(N$^a$-methyl)Arg-Gly-Asp-Pen-NH$_2$ or cyclo(S,S)-(2-mercapto)benzoyl-(N$^a$-methyl)Arg-Gly-Asp-(2-mercapto)phenylamide.

8. A microemulsion as claimed in any one of claims 1 to 7 in which the diameter of droplets or particles is less than 100 nm.

9. A microemulsion as claimed in any one of claims 1 to 7 in which the diameter of droplets or particles is in the range of 5 to 35 nm.

10. A microemulsion as claimed in any one of claims 1 to 9 in which the lipophilic phase comprises 10-95%, the high HLB surfactant from 5 to 90% and the hydrophilic phase less than 40%(w/w) of the microemulsion and in which the ratio of medium-chain fatty acid triglyceride to low HLB surfactant is between 4:1 and 2:1.

11. A microemulsion as claimed in claim 10 in which the lipophilic phase comprises 40 to 90%, the high HLB surfactant from 5 to 50% and the hydrophilic phase less than 10% (w/w) of the microemulsion.

12. A microemulsion as claimed in claim 12 in which the lipophilic phase comprises 60 to 90%, the high HLB surfactant from 5 to 30% and the hydrophilic phase less than 5% (w/w) of the microemulsion.

13. A self-emulsifying water-in-oil (w/o) microemulsion optionally comprising a water-soluble therapeutic agent in which the relative proportions of the following components:

(1) a lipophilic phase comprising a medium-chain fatty acid triglyceride or a blend of medium-chain fatty acid triglycerides, and a low HLB surfactant or blend of low HLB surfactants;
(2) a high HLB surfactant selected from a polyoxyethylene fatty acid ester, a polyoxyethylene-sorbitan fatty acid ester, a polyoxyethylene glycol long-chain alkyl ether or a polyoxyethylene glycol long-chain alkyl ester; and
(3) an aqueous phase;

lie within any one of the shaded regions (A), (B) and (C) of any of

a) figure 2 related to

$$\frac{\text{CAPTEX-335}}{\text{CAPMUL-MCM™}} = \frac{4}{1},$$

TWEEN-80 and water,
b) figure 3 related to

$$\frac{\text{CAPTEX-335}}{\text{CAPMUL-MCM™}} = \frac{3}{1},$$

TWEEN-80 and water,
c) figure 4 related to

$$\frac{\text{CAPTEX-335}}{\text{CAPMUL-MCM™}} = \frac{2}{1},$$

TWEEN-80 and water and
d) figure 5 related to

$$\frac{\text{CAPTEX-2000}}{\text{CAPMUL-C8}} = \frac{2}{1},$$

TWEEN-80 and water.

14. A microemulsion as claimed in claim 13 in which the relative proportions of components (1) to (3) lie within regions (A) and (B).

15. A microemulsion as claimed in any one of claims 1 to 14 adapted for oral delivery.

16. A microemulsion as claimed in any one of claims 1 to 14 adapted for topical delivery.

17. The use of a microemulsion according to any one of claims 1-15 comprising a medium-chain fatty acid triglyceride, a low HLB surfactant, a high HLB surfactant, a therapeutic agent and a hydrophilic phase in the manufacture of a medicament.

**Patentansprüche**

1. Pharmazeutisch verträgliche, stabile, selbstemulgierende Wasser-in-Öl (W/O)-Mikroemulsion, umfassend:

   (a) eine lipophile Phase, die ein Triglycerid mit Fettsäureketten mittlerer Länge und ein grenzflächenaktives Mittel mit einem niedrigen HLB-Wert aufweist, und worin das Verhältnis des Triglycerids mit Fettsäureketten mittlerer Länges zu dem grenzflächenaktiven Mittel mit niedrigen HLB-Wert 5:1 bis 1,5:1 ist;
   (b) ein grenzflächenaktives Mittel mit hohem HLB-Wert, ausgewählt aus einem Polyoxyethylenfettsäureester, einem Polyoxyethylensorbitanfettsäureester, einem Polyoxyethylenglykol-langkettigen Alkylether oder einem Polyoxyethylenglykol-langkettigen Alkylester; wobei das grenzflächenaktive Mittel in einem Bereich von 5 bis 75 % (Gew./Gew.) in der Mikroemulsion vorhanden ist;
   (c) eine wäßrige hydrophile Phase; und
   (d) ein wasserlösliches therapeutisches Mittel.

2. Mikroemulsion nach Anspruch 1, wobei das Triglycerid 50 bis 100 % (Gew./Gew.) Caprylsäure und 0 bis 50 % (Gew./Gew.) Caprinsäuretriglyceride umfaßt.

3. Mikroemulsion nach Anspruch 1 oder 2, wobei das grenzflächenaktive Mittel mit niedrigem HLB-Wert ein Monoglycerid oder Diglycerid mit Fettsäureketten mittlerer Länge oder ein Gemisch daraus ist, und gegebenenfalls eine geringe Gewichtsmenge von freien Fettsäuren mit Ketten mittlerer Länge umfaßt.

4. Mikroemulsion nach Anspruch 3, wobei das Monoglycerid oder Diglycerid mit Fettsäureketten mittlerer Länge aus etwa 50 bis 100 % Caprylsäure und aus etwa 0 bis 50 % Caprinsäure erzeugt wird.

5. Mikroemulsion nach einem der Ansprüche 1 bis 4, wobei das therapeutische Mittel ein Peptid mit einem Molekulargewicht im Bereich von 100 bis 10 000 ist.

6. Mikroemulsion nach Anspruch 5, wobei das Peptid ein RGD enthaltendes Peptid, das Peptid GHRP (His-D-Trp-Ala-Trp-D-Phe-Lys-NH$_2$), ein Fibrinogenrezeptor-antagonistisches Peptid (ein RGD-Peptid), ein Vasopressin, ein Calcitonin, oder ein Insulin ist.

7. Mikroemulsion nach Anspruch 6, wobei das RGD-Peptid Cyclo(S,S)-N$^a$-acetyl-Cys-(N$^a$-methyl)Arg-Gly-Asp-Pen-NH$_2$ oder Cyclo(S,S)-(2-mercapto)benzoyl-(N$^a$-methyl)Arg-Gly-Asp-(2-mercapto)phenylamid ist.

8. Mikroemulsion nach einem der Ansprüche 1 bis 7, wobei der Durchmesser von Tröpfchen oder Teilchen weniger als 100 nm beträgt.

9. Mikroemulsion nach einem der Ansprüche 1 bis 7, wobei der Durchmesser von Tröpfchen oder Teilchen im Bereich von 5 bis 35 nm liegt.

10. Mikroemulsion nach einem der Ansprüche 1 bis 9, wobei die lipophile Phase 10 bis 95 %, das grenzflächenaktive Mittel mit einem hohen HLB.Wert 5 bis 90 % und die hydrophile Phase weniger als 40 % (Gew./Gew.) der Mikroemulsion umfaßt und wobei das Verhältnis des Triglycerids mit Fettsäureketten mittlerer Länge zum grenzflächenaktiven Mittel mit niedrigem HLB-Wert zwischen 4:1 und 2:1 ist.

11. Mikroemulsion nach Anspruch 10, wobei die lipophile Phase 40 bis 90 %, das grenzflächenaktive Mittel mit hohem HLB-Wert 5 bis 50 % und die hydrophile Phase weniger als 10 % (Gew./Gew.) der Mikroemulsion umfaßt.

12. Mikroemulsion nach Anspruch 11, wobei die lipophile Phase 60 bis 90 %, das grenzflächenaktive Mittel mit hohem HLB-Wert 5 bis 30 % und die hydrophile Phase weniger als 5 % (Gew./Gew.) der Mikroemulsion umfaßt.

13. Selbstemulgierende Wasser-in-Öl (W/O)-Mikroemulsion, die gegebenenfalls ein wasserlösliches therapeutisches Mittel umfaßt, in der die relativen Anteile der folgenden Komponenten:

    (1) eine lipophile Phase, umfassend ein Triglycerid mit Fettsäureketten mittlerer Länge oder ein Gemisch aus Triglyceriden mit Fettsäureketten mittlerer Länge und ein grenzflächenaktives Mittel mit niedrigem HLB-Wert oder ein Gemisch aus grenzflächenaktiven Mitteln mit niedrigem HLB-Wert;

(2) ein grenzflächenaktives Mittel mit hohem HLB-Wert, ausgewählt aus einem Polyoxyethylenfettsäureester, einem Polyoxyethylensorbitanfettsäureester, einem Polyoxyethylenglykol-langkettigen Alkylether oder einem Polyoxyethylenglykol-langkettigen Alkylester; und

(3) eine wäßrige Phase;

innerhalb eines der schattierten Bereiche (A), (B) und (C) liegen von einem der

a) Figur 2 betreffend

$$\frac{\text{CAPTEX-335}}{\text{CAPMUL-MCM}^{\circledR}} = \frac{4}{1},$$

TWEEN-80 und Wasser

b) Figur 3 betreffend

$$\frac{\text{CAPTEX-335}}{\text{CAPMUL-MCM}^{\circledR}} = \frac{3}{1},$$

TWEEN-80 und Wasser

c) Figur 4 betreffend

$$\frac{\text{CAPTEX-335}}{\text{CAPMUL-MCM}^{\circledR}} = \frac{2}{1},$$

TWEEN-80 und Wasser, und

d) Figur 5 betreffend

$$\frac{\text{CAPTEX-2000}}{\text{CAPMUL-C8}} = \frac{2}{1},$$

TWEEN-80 und Wasser.

**14.** Mikroemulsion nach Anspruch 13, wobei die relativen Anteile der Komponenten (1) bis (3) in den Bereichen (A) und (B) liegen.

**15.** Mikroemulsion nach einem der Ansprüche 1 bis 14, die für die orale Verabreichung angepaßt ist.

**16.** Mikroemulsion nach einem der Ansprüche 1 bis 14, die für die örtliche Verabreichung angepaßt ist.

**17.** Verwendung einer Mikroemulsion nach einem der Ansprüche 1 bis 15, umfassend ein Triglycerid mit Fettsäureketten mittlerer Länge, ein grenzflächenaktives Mittel mit niedrigem HLB-Wert, ein grenzflächenaktives Mittel mit hohem HLB-Wert, ein therapeutisches Mittel und eine hydrophile Phase, für die Herstellung eines Medikaments.

**Revendications**

**1.** Microémulsion eau-dans-huile (e/h) autoémulsionnable, stable, acceptable du point de vue pharmaceutique, comprenant :

(a) une phase lipophile ayant un triglycéride d'acide gras à chaîne moyenne et un agent tensio-actif à faible équilibre hydrophile/lipophile et dans laquelle le rapport du triglycéride d'acide gras à chaîne moyenne à l'agent tensio-actif à faible équilibre hydrophile/lipophile va de 5:1 à 1,5:1 ;

(b) un agent tensio-actif à fort équilibre hydrophile/lipophile choisi entre un ester d'acide gras polyoxyéthylénique, un ester d'acide gras de polyoxyéthylènesorbitanne, un éther d'alkyle à longue chaîne de polyoxyéthylèneglycol ou un ester d'alkyle à longue chaîne de polyoxyéthylèneglycol ; cet agent tensio-actif étant présent dans la plage de 5 à 75 % (en poids/poids) de la microémulsion ;

(c) une phase aqueuse hydrophile ; et

(d) un agent thérapeutique soluble dans l'eau.

2. Microémulsion suivant la revendication 1, dans laquelle le triglycéride comprend 50 à 100 % (en poids/poids) de triglycérides d'acide caprylique et 0 à 50 % (en poids/poids) de triglycérides d'acide caprique.

3. Microémulsion suivant la revendication 1 ou 2, dans laquelle l'agent tensio-actif à faible équilibre hydrophile/lipophile est un monoglycéride ou un diglycéride d'acide gras à chaîne moyenne ou un mélange des deux, comprenant facultativement une petite quantité en poids d'acide gras libre à chaîne moyenne.

4. Microémulsion suivant la revendication 3, dans laquelle le monoglycéride ou le diglycéride d'acide gras à chaîne moyenne est formé d'environ 50 à 100 % d'acide caprylique et d'environ 0 à 50 % d'acide caprique.

5. Microémulsion suivant l'une quelconque des revendications 1 à 4, dans laquelle l'agent thérapeutique est un peptide ayant un poids moléculaire compris dans la plage de 100 à 10 000.

6. Microémulsion suivant la revendication 5, dans laquelle le peptide est un peptide contenant le RGD, le peptide GHRP (His-D-Trp-Ala-Trp-D-Phe-Lys-NH$_2$), un peptide antagoniste des récepteurs de fibrinogène (peptide RGD), une vasopressine, une calcitonine ou une insuline.

7. Microémulsion suivant la revendication 6, dans laquelle le peptide RGD est le cyclo(S,S)-N$^a$-acétyl-Cys-(N$^a$-méthyl)Arg-Gly-Asp-Pen-NH$_2$ ou le cyclo(S,S)-(2-mercapto)benzoyl-(N$^a$-méthyl)Arg-Gly-Asp-(2-mercapto)phénylamide.

8. Microémulsion suivant l'une quelconque des revendications 1 à 7, dans laquelle le diamètre des gouttelettes ou des particules est inférieur à 100 nm.

9. Microémulsion suivant l'une quelconque des revendications 1 à 7, dans laquelle le diamètre des gouttelettes ou des particules se situe dans la plage de 5 à 35 nm.

10. Microémulsion suivant l'une quelconque des revendications 1 à 9, dans laquelle la phase lipophile compte pour 10 à 95 %, l'agent tensio-actif à fort équilibre hydrophile/lipophile compte pour 5 à 90 % et la phase hydrophile compte pour moins de 40 % (en poids/poids) de la microémulsion et dans laquelle le rapport du triglycéride d'acide gras à chaîne moyenne à l'agent tensio-actif à faible équilibre hydrophile/lipophile est compris entre 4:1 et 2:1.

11. Microémulsion suivant la revendication 10, dans laquelle la phase lipophile compte pour 40 à 90 %, l'agent tensio-actif à fort équilibre hydrophile/lipophile compte pour 5 à 50 % et la phase hydrophile compte pour moins de 10 % (en poids/poids) de la microémulsion.

12. Microémulsion suivant la revendication 12, dans laquelle la phase lipophile compte pour 60 à 90 %, l'agent tensio-actif à fort équilibre hydrophile/lipophile compte pour 5 à 30 % et la phase hydrophile compte pour moins de 5 % (en poids/poids) de l'émulsion.

13. Microémulsion eau-dans-huile (e/h) autoémulsionnable comprenant facultativement un agent thérapeutique hydrosoluble, dans laquelle les proportions relatives des composants suivants :

(1) une phase lipophile comprenant un triglycéride d'acide gras à chaîne moyenne ou un mélange de triglycérides d'acides gras à chaîne moyenne et un agent tensio-actif à faible équilibre hydrophile/lipophile ou un mélange d'agents tensio-actifs à faible équilibre hydrophile/lipophile ;
(2) un agent tensio-actif à fort équilibre hydrophile/lipophile choisi entre un ester d'acide gras polyoxyéthylénique, un ester d'acide gras de polyéthylènesorbitanne, un éther d'alkyle à longue chaîne de polyoxyéthylèneglycol ou un ester d'alkyle à longue chaîne de polyoxyéthylèneglycol ; et
(3) une phase aqueuse ;

se situent dans l'une quelconque des régions ombrées (A), (B) et (C) de l'une quelconque des :

a) figure 2 relative à

$$\frac{\text{CAPTEX-335}}{\text{CAPMUL-MCM™}} = \frac{4}{1},$$

TWEEN-80 et eau,

b) figure 3 relative à

$$\frac{\text{CAPTEX-335}}{\text{CAPMUL-MCM}^{\text{TM}}} = \frac{3}{1},$$

TWEEN-80 et eau,

c) figure 4 relative à

$$\frac{\text{CAPTEX-335}}{\text{CAPMUL-MCM}^{\text{TM}}} = \frac{2}{1},$$

TWEEN-80 et eau, et

d) figure 5 relative à

$$\frac{\text{CAPTEX-2000}}{\text{CAPMUL-C8}} = \frac{2}{1},$$

TWEEN-80 et eau,

14. Microémulsion suivant la revendication 13, dans laquelle les proportions relatives des composants (1) à (3) se situent dans les régions (A) et (B).

15. Microémulsion suivant l'une quelconque des revendications 1 à 14, adaptée à la libération orale.

16. Microémulsion suivant l'une quelconque des revendications 1 à 14, adaptée à la libération topique.

17. Utilisation d'une microémulsion suivant l'une quelconque des revendications 1 à 15 comprenant un triglycéride d'acide gras à chaîne moyenne, un agent tensio-actif à faible équilibre hydrophile/lipophile, un agent tensio-actif à fort équilibre hydrophile/lipophile, un agent thérapeutique et une phase hydrophile, dans la préparation d'un médicament.

Figure 1

**Figure 2**

**Figure 3**

EP 0 597 007 B1

Figure 4

Figure 5